# EUROPEAN PATENT APPLICATION

(11) **EP 0 709 068 A2**
(43) Date of publication of application: **01.05.1996**
(21) Application number: 95307702.1
(22) Date of filing: 27.10.1995
(51) Int. Cl.: A61F 2/06

(54) **X-ray visible stent**

(30) Priority: 27.10.1994 US 330623
(71) Applicant: MEDINOL LTD., 51581 Tel Aviv (IL)
(72) Inventor: Pinchasik, Gregory, Ramat Hasharon 47414 (IL)
(74) Representative: Bayliss, Geoffrey Cyril

(57) **Abstract**

A stent having a significant amount of mass at least at one end (15) thereof is described. In one embodiment, the stent is plated with a material, such as gold or Tantalum, having a high atomic weight. The plating can cover the entire stent or just a portion thereof. In a second embodiment, the ends (15) of the stent include protrusions (22) having enough metal therein to make them X-ray visible.

## Description

The present invention relates to stents generally and to stents which are visible to X-ray radiation in particular.

Stents are known in the art. They are typically formed of a cylindrical metal mesh which can expand when pressure is internally applied. Alternatively, they can be formed of wire wrapped into a cylindrical shape. Typically, the metal mesh or wire is very thin, thereby to provide the stent with significant flexibility.

Stents are typically placed into clogged or partially clogged blood vessels for the purpose of expanding the blood vessels and, more importantly, for maintaining the blood vessels in the expanded state for a long time.

Even though the stents perform well, the blood vessel may begin to clog, albeit in a different location. At this point, it may be desired to place a second stent in the blood vessel. If the new clogging is near the location of the old stent, as is often the case, it is desirable to place the new stent right after the old one. This requires being able to view the old stent, typically while being illuminated by X-ray radiation. However, stents cannot be seen with X-ray radiation since they are so thin.

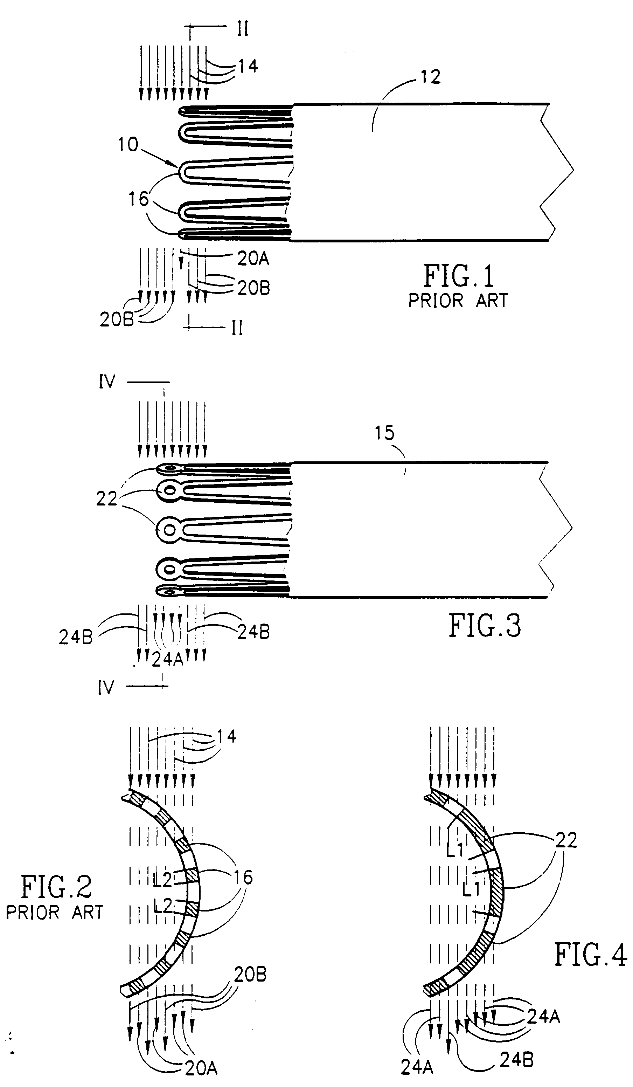

This is illustrated in Figures 1 and 2 to which reference is now briefly made. Figure 1 illustrates an end 10 of a stent 12 which is illuminated by X-rays 14. Figure 2 shows the cross-section, along lines II-II of Figure 1, of the stent 12, also being illuminated by the x-rays 14.

The stent 12 typically has expanded loops 16, typically of a thin metal. Thus, when stent 12 is illuminated by x-rays 14, most of the X-rays pass right through the loops 16. The output rays are labelled 20. Only those labelled 20a are absorbed by the loops 16. The remaining rays, labelled 20b, pass right through the stent 12. In Figure 1, only one ray of 9 is absorbed. In the cross-section of Figure 2, half of the rays are absorbed.

Because so few rays are absorbed by the loops 16, the stent 12 will not be visible in the film illuminated by the X-rays and thus, its exact location in the blood vessel cannot be determined.

It is therefore an object of the present invention to provide a stent which is X-ray visible.

Applicants have realised that elements having a significant amount of mass will be visible to X-ray illumination. In one embodiment, the stent is plated with a material, such as gold or Tantalum, having a high atomic weight such that mass of the stent will become significant to the X-ray illumination. The plating can cover the entire stent or just the ends.

In accordance with a second embodiment of the present invention, the ends of the stent include protrusions having enough metal therein to make them X-ray visible.

The present invention will be understood and appreciated more fully from the following detailed description taken in conjunction with the drawings in which:
Figure 1 is a schematic illustration of a prior art stent being illuminated by X-rays;
Figure 2 is a cross-section view, along lines II-II of Figure 1, of the prior art stent of Figure 1 being illuminated by x-rays;
Figure 3 is a schematic illustration of a stent, constructed and operative in accordance with a preferred embodiment of the present invention, being illuminated by X-rays;
Figure 4 is a cross-section view, along lines IV-IV of Figure 3, of the stent of Figure 3 being illuminated by X-rays.

Applicants have realised that elements having a significant amount of mass will be visible to X-ray illumination.

Since stents are typically formed of metal, they can be plated. In accordance with one embodiment of the present invention, the stent is plated with a material having a high atomic weight such that mass of the stent will become significant to the X-ray illumination. The plating can cover the entire stent or just the edges.

Exemplary plating materials are gold (atomic weight 197,200) and Tantalum (atomic weight 180,880) whose atomic weights are considerably larger than that of steel (atomic weight 55.80) or stainless steel (weight slightly larger than that of steel), both of which are common materials for stents. As can be seen, for the same amount of material, gold and Tantalum are much heavier and thus, will be more visible during X-ray illumination.

It is noted that the interaction of the plating material, the metal of the stent and the blood may induce an electrical current between the metal of the stent and the plating material. However, the amount of current is a function of the metal of the stent, the type of plating material and their amounts and thus, can be made to be small.

In accordance with a second embodiment of the present invention and as illustrated in Figures 3 and 4 to which reference is now made, the ends of the stent, labelled 15, are formed with protrusions 22 having enough metal therein to make them X-ray visible.

As illustrated in Figure 4, protrusions 22 have a width L1 whereas, as shown in Figure 2, the loops 16 have a width L2. The protrusion width L1 is typically 2 - 5 times as large as that of the loops, thereby to provide 2 - 5 times as much material in the protrusions 22 as in the end portions of the loops 16.

As can be seen from Figures 3 and 4, the protrusions 22 absorb most of the X-rays 14. Thus, many of the X-rays 14 are absorbed and there are fewer, and less strong, exiting rays, labelled 24. Thus, in Figure 3, four X-rays 24a are partially absorbed by the protrusions 20 while five X-rays 24b are not affected. In figure 4, only one X-ray 24b is not affected. The remaining x-rays 24a are partially or almost completely absorbed.

It will be appreciated that the number of absorbed X-rays 24a is larger for the stent 15 of Figure 3 than for the prior art stent of Figure 1, due to the extra material in the protrusions 22. Thus, the stent 15 is generally more x-ray visible than is the prior art stent 12 of Figure 1.

It will be appreciated by persons skilled in the art that the present invention is not limited to what has been particularly shown and described hereinabove. Rather the scope of the present invention is defined only by the claims which follow:

## Claims

1. An X-ray visible stent formed of a body having a significant amount of mass at least at one end thereof.

2. An x-ray visible stent formed of a body formed from a first type of metal at least a portion of which is plated with a second type of metal, wherein said second type of metal has a high atomic weight.

3. An X-ray visible stent formed of a thin metal body having metal protrusions at at least one edge thereof.

4. A stent according to claim 3 and wherein said edges have loops of a first width and said protrusions have a second width which is 2 to 5 times as wide as said first width.
